# EUROPÄISCHE PATENTSCHRIFT

(11) **EP 1 275 426 B1**
(45) Veröffentlichungstag und Bekanntmachung des Hinweises auf die Patenterteilung: **06.12.2006**
(21) Anmeldenummer: 02013751.9
(22) Anmeldetag: 21.06.2002
(51) Int. Cl.: B01D 19/04, A61M 1/16, A61M 1/36

(54) **Vorrichtung mit einer mit einem Entschäumungsmittel beschichteten Oberfläche und Verwendung einer Oberfläche mit einem Entschäumungsmittel**
Device comprising a surface coated with a defoaming agent and use of a surface coated with a defoaming agent
Dispositif avec une surface revêtue d'un agent antimousse et usage d'une surface avec un agent antimousse

(30) Priorität: 13.07.2001 DE 10135277
(43) Veröffentlichungstag der Anmeldung: 15.01.2003
(73) Patentinhaber: Maquet Cardiopulmonary AG, 72145 Hirrlingen (DE)
(72) Erfinder: Sevastianov, Viktor I., Prof. Dr., Moskau 123182 (RU)
(74) Vertreter: Otten, Hajo

(56) Entgegenhaltungen:
- EP-A- 0 630 656
- US-A- 4 597 894
- US-A- 5 543 082
- US-A- 5 582 794

## Beschreibung

Die Erfindung betrifft eine Vorrichtung zum Entschäumen von Blut mit einer mit Blut in Kontakt kommenden Oberfläche, die mit einem Entschäumungsmittel beschichtet ist, sowie ein derartiges Entschäumungsmittel.

Die Erfindung betrifft weiterhin eine Verwendung einer mit Blut in Kontakt kommenden Oberfläche in einem extrakorporalen Kreislauf zum Entschäumen von Blut.

Eine Vorrichtung der vorstehend genannten Art ist aus der EP 0 774 285 bekannt.

Derartige Vorrichtungen werden z.B. bei herzchirurgischen Eingriffen für das Entschäumen von Blut eingesetzt. Bei Operationen am offenen Herzen werden die Funktionen von Herz und Lunge des Patienten in einem extrakorporalen Blutkreislauf mit Hilfe von Pumpen und Oxygenatoren von einer Herz-Lungen-Maschine übernommen. Dabei wird Blut, insbesondere das aus einer Operationswunde abgesaugte Blut, oftmals unter Schaumbildung mit Luft vermengt. Dieser Schaum bzw. die Schaumbläschen müssen aus dem Blut entfernt werden, bevor das Blut dem Patienten zurückgeführt wird, da dieser ansonsten der Gefahr von Embolien ausgesetzt ist.

So wird z.B. an ein extrakorporales System, bestehend aus einem Oxygenator, Blut- und Cardiotomie-Reservoiren sowie Blutschläuchen und Filtern usw., die Anforderung gestellt, dem Blut nicht nur Sauerstoff zu- und CO₂ abzuführen, sondern auch den durch Bläschenbildung entstandenen Schaum wieder vollständig zu entfernen. Blutfilter und andere Vorrichtungen, wie z.B. Blasenfallen, Abscheide-Zyklone o.ä., die in einen extrakorporalen Blutkreislauf eingeschaltet werden, können den Einsatz von Entschäumern im Blutreservoir und vor allem im Cardiotomiereservoir nicht ersetzen.

Ein Entschäumen wird normalerweise dadurch erzielt, daß das Blut mit einer sehr großen, hydrophoben Oberfläche in Kontakt gebracht wird, die mit einem entschäumenden Mittel beschichtet ist. Als Oberfläche werden hier insbesondere offenzellige Polyurethanschäume oder textile Materialien z.B. aus Polyester eingesetzt.

Die Oberflächen werden mit Verbindungen beschichtet, die an der Grenzfläche zwischen flüssig und gasförmig einen geschlossenen Film bilden und es dadurch der zu entgasenden Flüssigkeit ermöglichen, die kleinste Oberfläche auszubilden. Durch dieses "Entschäumungsmittel" werden die Gasbläschen zerstört.

Der weitaus am häufigsten verwendete Entschäumer zur Beschichtung von mit Blut in Kontakt kommenden Oberflächen ist Siliconöl (Polydimethylsiloxan) oder eine Mischung aus Polydimethylsiloxan und Silicon-Dioxid, welche unter dem Handelsnamen "Simethicon®" und "Antifoam A®" von Dow Corning vertrieben werden.

Diese Entschäumungsmittel besitzen den Nachteil, daß sie vom strömenden Blut langsam von der Oberfläche abgewaschen und somit in ihrer Wirksamkeit gemindert werden. Dadurch wird das Blut nach einiger Zeit nicht mehr ausreichend entschäumt. Die nicht aus dem Blut entfernten Gasbläschen können im Patienten Embolien hervorrufen. Außerdem bedeutet das Auswaschen, daß Siliconöl und Silica-Partikel bei langwierigen Operationen in den Patienten gelangen und dort ebenfalls Embolien auslösen können. Diese Gefahr wird weiterhin dadurch verstärkt, daß heute vorwiegend in ihrer Hämokompatibilität verbesserte Materialien für den extrakorporalen Blutkreislauf eingesetzt werden. Diese besitzen dann beispielsweise aufgrund einer Heparinisierung eine hydrophile Oberfläche, so daß abgewaschenes Siliconöl nicht mehr durch hydrophobe Wechselwirkungen von hydrophoben Oberflächen im extrakorporalen Blutkreislauf vor dem Eintritt in den Patienten abgefangen werden kann.

Aus der eingangs genannten EP 0 774 285 ist es bekannt, eine hydrophobe Oberfläche mit einem Entschäumungsmittel zu beschichten, das ein Triglycerid mit mindestens einer Fettsäure aufweist, die 14 bis 24 Kohlenstoffatome enthält. Vorzugsweise wird hierbei Rizinusöl verwendet. Dabei kann das Entschäumungsmittel auch noch einen hydrophoben Bestandteil, bspw. eine Siliconverbindung, aufweisen.

Dieser Entschäumer erwies sich jedoch im Vergleich mit beispielsweise Simethicon® als weitaus weniger wirksam.

Außerdem zeigte sich, daß auch bei einem Rizinusöl enthaltenden Entschäumer - wie bei nur mit Silicon beschichteten Oberflächen - das Öl bei einer länger andauernden Benutzung des Entschäumers durch das strömende Blut abgewaschen wird, in den Patienten gelangt und dort eine Fettembolie auslösen kann.

Die US 5,582,794 beschreibt eine medizinische Vorrichtung mit einer mit Blut in Kontakt kommenden Oberfläche, welche mit den Polymeren Hydroxyethylmethacrylat, Methylmethacrylat und Poly(oxyethylen)-poly(oxypropylen) beschichtet ist.

Die EP 0 630 656 offenbart medizinische Vorrichtungen, die eine mit Blut in Kontakt kommende Oberfläche aufweisen, welche mit einem biokompatiblen, oberflächenaktiven Entschäumungsmittel beschichtet ist, das ein primär alkoxylierter Alkohol ist.

Die US 4.597.894 offenbart eine Silicon-basierte Entschäumungs-Zusammensetzung, die als Block-Copolymer partiell fluorierte Alkyl-Seitengruppen aufweist. Diese Entschäumer-Zusammensetzung ist selbst-emulgierend im Wasser. Die sehr stabile wässrige Emulsion dient - vorzugsweise mit Silica-Partikeln zusammen - zur Entschäumung technischer Flüssigkeiten. Als Oberflächenbeschichtung erwies sich dieses Block-Copolymer allein als unwirksam. Silica-Partikel sollen für die medizinische Anwendung vermieden werden.

In der WO 95/28184 wird eine Beschichtung von Polymeren oder Metallen offenbart, die Triblock-Copolymere aus Polylacton-Polysiloxan-Polylacton aufweist, wobei das Siloxan DimethylSiloxan und das Lacton Caprolacton ist. Diese Beschichtung zeigte sich als besonders biokompatibel und geeignet für den Einsatz in extrakorporalen Blutkreisläufen, da hier das Blut mit einer besonders großen Oberfläche in Kontakt kommt. Diese Beschichtung kann nicht nur auf poröse Membranen, sondern auch auf Metalloberflächen stabil aufgetragen werden, wodurch sie biokompatibel wurden.

Für den Einsatz als Entschäumer erwies sich die Beschichtung mit den Triblock-Copolymeren allerdings als unwirksam.

Vor diesem Hintergrund ist es daher Aufgabe der vorliegenden Erfindung, eine Vorrichtung der eingangs genannten Art zu schaffen, die mit einem dauerhaften und gleichzeitig in seiner Wirksamkeit nicht mit der Zeit herabgesetzten Entschäumungsmittel beschichtet ist.

Erfindungsgemäß wird diese Aufgabe dadurch gelöst, dass das Entschäumungsmittel aus einer Silicon-Verbindung mit Fluor enthaltenden lipophoben Mikrodomänen aufgebaut ist.

Diese Aufgabe wird weiterhin durch die Verwendung einer mit einem Entschäumungsmittel beschichteten Oberfläche gelöst, wobei das Entschäumungsmittel aus einer Silicon-Verbindung mit Fluor enthaltenden lipophoben Mikrodomänen aufgebaut ist.

Der Erfinder hat nämlich erkannt, dass mit dem Einsatz von hydrophob-lipophoben Materialien als Beschichtungsmittel eine mit Simethicon® vergleichbare Entschäumungswirkung erzielbar ist und gleichzeitig die Beständigkeit und die Blutverträglichkeit der Beschichtung erhöht werden kann.

Silicon-Verbindungen haben sich als Beschichtungsmaterial für mit Blut in Kontakt kommende Oberfläche bewährt und zeichnen sich durch eine sehr hohe Biokompatibilität aus. Als geeignet erwiesen sich dabei insbesondere Silicon-Copolymere, die eine Dimethylsiloxan-Komponente mit einem Molekulargewicht von ca. 1800 bis 2600 Da besitzen.

Bei der erfindungsgemäßen Vorrichtung ist weiterhin bevorzugt, wenn das Entschäumungsmittel durch eine Reaktion einer Silicon-Verbindung mit einer fluorierten Dicarbonsäure oder mit einem fluorierten Dicarbonsäurederivat hergestellt ist.

Dabei ist bevorzugt, wenn das fluorierte Dicarbonsäurederivat Tetrafluoro-Bernsteinsäure-Diethylester ist.

Der Erfinder hat nämlich erkannt, dass in einer Fluor-haltigen hydrophoben Beschichtung überraschenderweise lipophobe Mikrodomänen ausbildet werden. Über diese lipophoben Stellen werden die Luftbläschen im Blut gebunden und das Blut in Verbindung mit der hydrophoben Komponente entschäumt. Insbesondere durch die Ausbildung von lipophoben Mikrodomänen ergibt sich eine gute Biokompatibilität.

Eine Beschichtung mit Fluor-haltigem Silicon erwies sich bei einem Einsatz in intravasculären Stents zwar als recht antithrombogen und biokompatibel (T. Matsuhashi et al.: In vivo Evalutaion of a Fluorine-Acryl-Stylene-Urethane-Silicone Antithrombogenic Coating Material Copolymer for Intravascular Stents; Acad. Radiol. 1996; 3: 581 - 588), die dort verwendete Beschichtungszusammensetzung zeigte aber keine Entschäumungswirkung. Sie wird bei der dort beschriebenen Anwendung allerdings auch nicht erwartet.

In einer weiter bevorzugten Ausführung zeigt das Entschäumungsmittel die allgemeine Formel

{R'N-R-Si(CH₃)₂-[OSi(CH₃)₂]ₙ-R-(NR')-(CO)-(CF₂)ₖ-CO-}ₘ

in der
- R: eine Alkylen- oder Aralkylengruppe,
- n: 5 bis 40, vorzugsweise 10 bis 35,
- R': ein Wasserstoffatom oder eine Alkyl- oder Aralkylgruppe,
- k: 2 bis 5 und
- m: eine ganze Zahl zwischen 1 und 15
ist.

Die mit dem erfindungsgemäßen Entschäumungsmittel zu beschichtende Oberfläche ist dabei aus einer Gruppe ausgewählt, umfassend hydrophobe Materialien wie Polyurethan, Polyester und andere Polymere.

Offenzellige Polyurethan-Schäume haben sich bei Vorrichtungen für Eingriffe am offenen Herzen bewährt und werden bei extrakorporalen Blutkreisläufen am häufigsten eingesetzt.

Dabei ist nicht ausgeschlossen, daß auch andere Materialien mit dem erfindungsgemäßen Entschäumungsmittel beschichtet werden können.

In einer Ausführungsform ist die erfindungsgemäße Vorrichtung als in einen extrakorporalen Kreislauf einschaltbarer Filter ausgebildet.

Weiterhin ist bevorzugt, wenn die Vorrichtung mit der erfindungsgemäß beschichteten Oberfläche als Entschäumungsfilter in einem Blutreservoir ausgebildet ist.

Als Blutreservoir können Weichbeutelreservoire und/oder Hartschalenreservoire gängiger Formgebung verwendet werden.

Weitere Vorteile ergeben sich aus der Beschreibung, der Zeichnung und den Beispielen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Es zeigen:
- Fig. 1: eine schematische Darstellung eines extrakorporalen Kreislaufs in Form einer Herz-Lungen-Maschine
- Fig. 2: einen stark vergrößerten, schematischen Ausschnitt einer erfindungsgemäß beschichteten Oberfläche

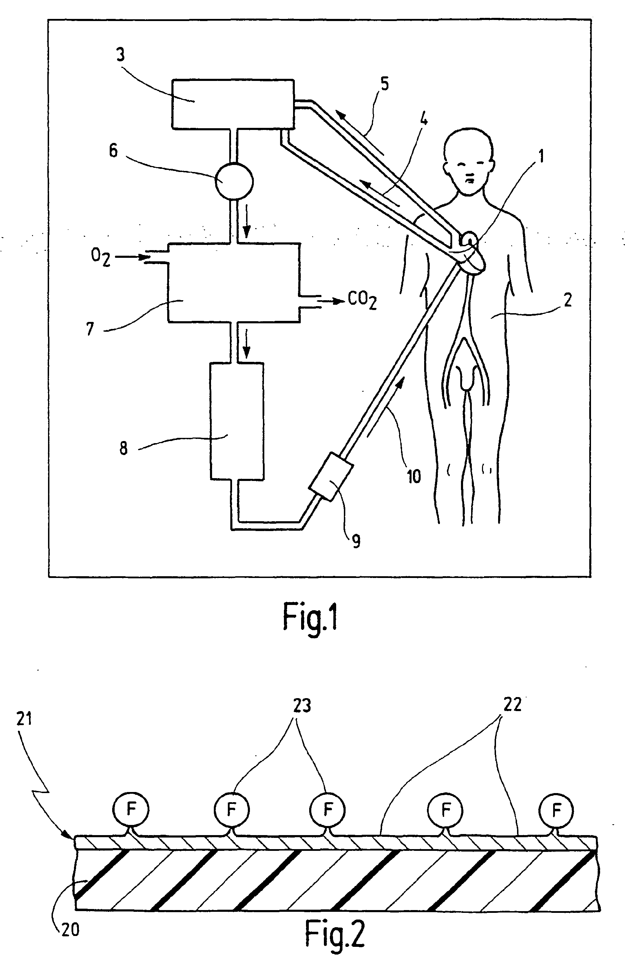

In Figur 1 ist eine Herz-Lungen-Maschine als Schema eines extrakorporalen Kreislaufs dargestellt. Die Herz-Lungen-Maschine übernimmt vorübergehend die Pumpfunktion des Herzens und die Funktion des Gasaustausches der Lungen.

Über ein Schlauchsystem 1 wird der rechte Vorhof des Patienten 2 mit der Herz-Lungen-Maschine verbunden. Venöses Blut fließt in ein Blutreservoir 3, was mit dem Pfeil 4 angedeutet ist. Dort wird es mit Blut vereint, das über Schläuche aus einer Operationswunde abgesaugt wird, was durch den Pfeil 5 gezeigt ist. Das Blutreservoir ist mit einer mit einem Entschäumungsmittel beschichteten Oberfläche versehen.

Über eine Pumpe 6 wird das vermengte Blut in einen Gasaustauscher (Oxygenator) 7 befördert, wo das Blut mit Sauerstoff angereichert und CO₂ aus dem Blut entfernt wird. Nach der Oxygenierung des Blutes wird dieses über einen Wärmeaustauscher 8 geführt, durch den das Blut und somit der Patient auf bestimmte Temperaturen abgekühlt bzw. aufgewärmt werden kann. Der Wärmeaustauscher 8 ist normalerweise integraler Bestandteil des Oxygenators. Über einen Filter 9 wird "arterialisiertes" Blut dem Patienten 2 wieder zurückgeführt, was mit dem Pfeil 10 angedeutet ist.

Daneben besteht aber auch die Möglichkeit, andere in einem extrakorporalen Kreislauf bereits vorhandene oder einschaltbare Komponenten mit der erfindungsgemäßen Beschichtung zu versehen. So können der Oxygenator oder etwa Filter derart beschichtet sein, daß sie nicht nur ihre jeweilige ursprüngliche Funktion, also die Sauerstoffzufuhr oder das Abfangen von Blutgerinnseln o.ä., sondern auch gleichzeitig eine entschäumende Wirkung besitzen. Voraussetzung für diese zusätzliche Funktion ist eine Möglichkeit, die gesammelte Luft abzuführen.

In Figur 2 ist ein stark vergrößerter Ausschnitt einer erfindungsgemäß beschichteten Oberfläche schematisch dargestellt. Dabei ist mit 20 eine Oberfläche bezeichnet, auf die ein Entschäumungsmittel 21 aufgebracht ist. Dieses Entschäumungsmittel 21 besitzt hydrophobe Domänen 22 und weist durch eine Fluorhaltige Komponente ausgebildete lipophobe Mikrodomänen 23 auf. Als Fluor-haltige Komponente kommt bspw. eine fluorhaltige Carbonsäure in Betracht, wobei das darin enthaltene Fluor lipophobe Mikrodomänen innerhalb einer durch eine Silicon-Verbindung ausgebildeten hydrophoben Schicht ausbildet.

Beispiele einer erfindungsgemäßen Beschichtung und deren Herstellung sind nachstehend ausführlich erläutert.

### Beispiele

A) Synthese von Tetrafluoro-Bernsteinsäurediethylester
Tetrafluoro-Bernsteinsäurediethylester wurde durch die Reaktion von Tetrafluoro-Bernsteinsäure mit einer überschüssigen Menge an Ethanol in Anwesenheit von Schwefelsäure als Katalysator der Esterifizierung bei 60-75 °C gewonnen. Nach Beendigung der Reaktion wird das Endprodukt Tetrafluoro-Bernsteinsäurediethylester (TFSDE) durch Destillation bei 185 °C isoliert.
B) Synthese des Entschäumungsmittels
Um das Entschäumungsmittel herzustellen, wurden 5,2 g (2 x 10⁻³ mol) Tegomer A-Si2322 (Goldschmidt AG, Deutschland), ein Silicon-Oliogmer mit terminalen sekundären Aminogruppen, mit einem Molekulargewicht von 2600 Da in 7 ml Isopropanol in einen Kolben gegeben, ausgestattet mit einem Magnetrührer und einem Thermometer und durch eine Vorlage gegen Luftfeuchtigkeit geschützt. 0,55 g (2,2 x 10⁻³ mol) Tetrafluoro-Bernsteinsäurediethylester (TFSDE, aus A) in 4,5 ml Isopropanol wurde tropfenweise und unter 5 min. Rühren bei 20 °C hinzugefügt. Die Temperatur der reagierenden Lösung wurde schrittweise erhöht: 20 °C für 0,5 h; 40 °C für 1 h; 60 °C für 0,5 h und 75 °C für 0,5 h. Nach Beendigung der Reaktion wurde der Kolben abgekühlt. Die erhaltene Lösung wurde unter Luftfeuchtigkeitsausschluß bei 5 °C gehalten und ohne zusätzliche Reinigungsschritte verwendet.
C) Beschichtungs-Vorgehensweise
Die Arbeitslösung enthielt 0,75 bis 1 Gew.-% der unter B) hergestellten Substanz, 95 ml Isopropanol und 5 ml an Novec® HFE7100 (3M, Deutschland). Proben von Polyurethan-schwämmen wurden mit der Arbeitslösung für 10 min bei Raumtemperatur inkubiert. Nach der Inkubation wurden die Proben vollständig bei 50 ± 5 °C für 1 bis 2 h getrocknet und anschließend durch Ethylenoxid sterilisiert.
D) Effizienz der Beschichtung
Zum Vergleich der Effizienz wurde in eine 10 %-ige Lösung von humanem Albumin Luft eingerührt und die entstehende Schaumlösung über gemäß C vorbereitete beschichtete Polyurethan-Schäume rezirkuliert. Zu Beginn des Versuches war die Entschäumungwirkung von nur mit Simethicon® und von erfindungsgemäß beschichteten Polyurethan-Schwämmen gleich, jedoch fiel für nur mit Simethicon® beschichtete Proben die Entschäumungswirkung im Verlauf von 20 - 30 min stark ab. Die Wirksamkeit der erfindungsgemäß beschichteten Proben blieb über Stunden konstant.
E) Hydrophobie und Biologische Eigenschaften der beschichteten Schwämme
Zum Test der Hydrophobizität der beschichteten Schwämme aus C wurden diese in destilliertem Wasser für 1 h ohne jegliche Luftblasen inkubiert. Anschließend wurden die Proben auf einer Höhe von 2 m auf die Unterlagen fallengelassen. Danach wurde das Gewicht der Proben gemessen. Der Wert der Wasseraufnahme wurde als das Verhältnis des Probengewichts mit Wasser zum Gewicht der trockenen Probe (g H₂O/g trockener Schwamm) definiert. Um die Beschichtungsstabilität zu bestimmen, wurde der Wert der Wasseraufnahme auch nach Inkubation der Proben für 20 h in destilliertem Wasser gemessen.
Die nachstehende Tabelle zeigt die zusammengefaßten Ergebnisse dieser Tests.

| Wasseraufnahme (g H₂O/g PU-Schwamm)* | unbeschichteter PU-Schwamm | Simethicon® beschichtet | erfindungsgemäß beschichtet |
|---|---|---|---|
| nach 1 h | 8,1 ± 1,0 | 5,6 ± 0,8 | 4,3 ± 0,5 |
| nach 24 h | nicht bestimmt | 7,2 ± 0,9 | 4,6 ± 0,5 |

| | | | |
|---|---|---|---|
| * PU-Schwamm = Polyurethan-Schwamm | | | |

Es zeigte sich, daß die erfindungsgemäß beschichteten Polyurethan-Schwämme eine deutlich höhere Hydrophobie aufweisen als die mit Simethicon® beschichteten. Dies zeigte sich besonders eindrucksvoll mit den Wert nach 24 h: Die mit Simethicon® beschichteten Schwämme ließen in ihrer Hydrophobie deutlich nach, nahmen also deutlich mehr Wasser auf (5,6 g nach 1 h im Vergleich zu 7,2 g nach 24 h pro g Polyurethan-Schwamm). Dagegen blieb der Wert der Wasseraufnahme für die erfindungsgemäß beschichteten nahezu konstant (4,3 g nach 1 h im Vergleich zu 4,6 g nach 24 h pro g Polyurethan-Schwamm).
Die Hämokompatibilität der Beschichtungen wurde anhand der Hämolyse, der Plasmarekalzifizierungszeit, der Adsorption von ¹³¹Jod-markiertem humanen Serumalbumin, dem Aussehen adhärierender Blutplättchen und der Komplementaktivierung untersucht.
Zum Test der Hämolyse wurde ein Extrakt des Beschichtungsmaterials in physiologischer Kochsalzlösung zubereitet und mit Blut für 1 h bei 37 °C inkubiert. Das Beschichtungsmaterial wurde entfernt und das Blut für 15 min bei 400 x g zentrifugiert. Der zellfreie Überstand wurde sorgfältig entfernt; der Grad der Hämolyse der Proben wurde durch spektrophometrische Messung bei einer Wellenlänge von 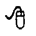 = 530 bis 550 nm gemessen.
Zur Bestimmung der Plasmarekalzifizierungszeit wurden die unter C vorbereiteten beschichteten Polyurethan-Schwämme mit Citrat-haltigem humanen Plasma für 20 min bei 37 °C inkubiert. Anschließend wurden dem Plasma 0,025 M Calciumchlorid hinzugefügt. Die Plasmarekalzifizierungszeit wurde bei 37 °C anhand des "Fibrintimer II" (Behring, Deutschland) bestimmt. Als Kontrolle diente dabei nichtinkubiertes Plasma.
Die Adsorption von ¹³¹Jod-markiertem humanen Serumalbumin an die innere Oberfläche der Proben wurde unter Verwendung eines Copra-5005 Gammazählers (Canberra-Packard, USA) gemessen. Die Gesamtaktivität des ¹³¹Jod-markierten humanen Serumalbumin (J-HSA) in phosphatgepufferter Saline (PBS) war nicht weniger als 100 cpm/mg min bei einer Gesamtvolumenkonzentration von HSA von 30 mg/ml. Nach einer Inkubation von 2 h bei Raumtemperatur in J-HSA-PBS wurden die Proben in einer physiologischen Kochsalzlösung mit einem pH von 7,4 gewaschen, und anschließend die Radioaktivität in einem Gammazähler gemessen.
Zur Überprüfung der adhärierenden Thrombozytenzahl wurden die Proben mit Plasma, angereichert mit Thrombozyten, inkubiert und anschließend die Proben mikroskopisch untersucht, wobei bei jeder Probe 400 µm² zufällig ausgesucht wurden.
Die Gesamtzahl der Thrombozyten wurde in drei Klassen unterteilt: in Einzelzellen, in deformierte Zellen und in Aggragate aus zwei oder mehr Zellen. Diese Klasseneinteilung gibt die Thrombozyten-Aktivierung wieder: Je geringer der Wert der Adhäsion der Thrombozyten, desto höher die Wahrscheinlichkeit der Biokompatibilität des getesteten Materials.
Die Komplementaktivierung vor und nach der Inkubation des humanen Plasmas mit den erfindungsgemäßen Beschichtungsmaterialien wurde anhand der photokolorimetrischen Methode bestimmt. Die Hämoglobinkonzentration, freigesetzt durch die lysierende Komplementwirkung aus mit Antikörpern beschichteten Schaf-Erythrozyten, gibt die Komplementaktivität in der Serumprobe wieder.
Der Test der Hydrophobizität und Stabilität der Beschichtungsmaterialien und die Bestimmung der biologischen Eigenschaften sind in der nachstehenden Tabelle zusammengefaßt.

| | Kontroll-Plasma bzw. Serum | unbeschich teter PU-Schaum | Silicon beschichtet | Erfindungs gemäß beschichtet |
|---|---|---|---|---|
| Hämolyse (%) | - | 0 | 0 | 0 |
| Plasmakalzifizierungszeit (s) | 294 ± 23 | 225 ± 25 | 250 ± 12 | 290 ± 15 |
| 131 J-HSA Adsorption (µg/g) | - | 11,6 ± 2,3 | 6,8 ± 1,2 | 13,4 ± 2,6 |
| Thrombozyten (REM) | - | - | Einzelzellen | Einzelzellen |
| Komplement aktivierung CH50 (%) | 95 ± 5 | - | 35 ± 2 | 50 ± 3 |

Hämolyse wurde weder von unbeschichteten noch von beschichteten Schwämmen ausgelöst. Aus der Tabelle ist ersichtlich, daß die erfindungsgemäß beschichteten Polyurethanschwämme das Blutgerinnungssystem praktisch nicht aktivierten, d.h. die Zeit, innerhalb derer das Blut gerinnt, ist bei diesen Proben nahezu gleich der Zeit, innerhalb derer Blut gerinnt, das nicht in Kontakt mit Schwämmen gebracht wurde. Im Vergleich dazu zeigten mit Silicon-beschichtete Schwämme eine deutlich schnellere Aktivierung des Blutgerinnungssystems ("Plasmarekalzifizierungszeit"): 250 ± 12 sec im Vergleich zu 294 ± 23 sec für unbehandeltes Plasma.
Weiterhin wurde das Komplementsystem durch die erfindungsgemäß beschichteten Polyurethanschwämme deutlich weniger aktiviert als durch die Silicon-beschichteten Schwämme. Gleichwohl unterschieden sie sich nicht von diesen in Bezug auf die Blutplättchenadhäsion.
Die erfindungsgemäß beschichteten Polyurethanschwämme adsorbierten 2 x mehr Albumin als Silicon-beschichtete Polyurethanschwämme (13,4 ± 2,6 µg/g gegenüber 6,8 ± 1,2 pg/g) und adsorbierten praktisch genauso viel Albumin wie ein unbeschichteter Schwamm (11,6 ± 2,3 µg/g).

## Patentansprüche

1. Vorrichtung zum Entschäumen von Blut mit einer mit Blut in Kontakt kommenden Oberfläche, die mit einem Entschäumungsmittel beschichtet ist, **dadurch gekennzeichnet, dass** das Entschäumungsmittel aus einer Silicon-Verbindung mit Fluor enthaltenden lipophoben Mikrodomänen aufgebaut ist.

2. Vorrichtung nach Anspruch 1, **dadurch gekennzeichnet, dass** das Entschäumungsmittel durch Reaktion einer Silicon-Verbindung mit einer fluorierten Dicarbonsäure oder mit einem fluorierten Dicarbonsäurederivat hergestellt ist.

3. Vorrichtung nach Anspruch 2, **dadurch gekennzeichnet, dass** das fluorierte Dicarbonsäurederivat Tetrafluoro-Bernsteinsäure-Diethylester ist.

4. Vorrichtung nach einem der Ansprüche 1 bis 3, **dadurch gekennzeichnet, dass** ein Entschäumungsmittel der allgemeinen Formel:
{R'N-R-Si(CH₃)₂-[OSi(CH₃)₂]ₙ-R-(NR')-(CO)-(CF₂)ₖ-CO-}ₙ
verwendet wird, in der
R eine Alkylen- oder Aralkylgruppe,
n 5 bis 40, vorzugsweise 10 bis 35,
R' ein Wasserstoffatom, eine Alkyl- oder Aralkylgruppe,
k 2 bis 5 und
m eine ganze Zahl zwischen 1 und 15
ist.

5. Vorrichtung nach einem der Ansprüche 1 bis 4, **dadurch gekennzeichnet, dass** die mit dem Entschäumungsmittel zu beschichtende Oberfläche hydrophobe Materialien umfasst, die aus der Gruppe umfassend Polyurethan, Polyester und andere Polymere, ausgewählt sind.

6. Vorrichtung nach einem der Ansprüche 1 bis 5, **dadurch gekennzeichnet, dass** sie als in einen extrakorporalen Kreislauf einschaltbarer Filter ausgebildet ist.

7. Vorrichtung nach einem der Ansprüche 1 bis 5, **dadurch gekennzeichnet, dass** sie als Entschäumungsfilter in einem Blutreservoir ausgebildet ist.

8. Verwendung einer mit einem Entschäumungsmittel beschichteten Oberfläche in einem extrakorporalen Kreislauf, wobei das Entschäumungsmittel aus einer Silicon-Verbindung mit Fluor enthaltenden lipophoben Mikrodomänen aufgebaut ist.

9. Entschäumungsmittel aus einer Silicon-Verbindung mit Fluor enthaltenden lipophoben Mikrodomänen, die fluorierte Dicarbonsäuren oder fluorierte Dicarbonsäurederivate enthalten.

10. Entschäumungsmittel nach Anspruch 9, **dadurch gekennzeichnet, dass** das fluorierte Dicarbonsäurederivat Tetrafluoro-Bernsteinsäure-Diethylester ist.

11. Entschäumungsmittel nach Anspruch 9 oder 10, mit der allgemeinen Formel
{R'N-R-Si(CH₃)₂-[OSi(CH₃)₂]ₙ-R-(NR')-(CO)-(CF₂)ₖ-CO-}ₘ
in der
R eine Alkylen- oder Aralkylengruppe,
n 5 bis 40, vorzugsweise 10 bis 35,
R' ein Wasserstoffatom, eine Alkyl- oder Aralkylgruppe,
k 2 bis 5 und
m eine ganze Zahl zwischen 1 und 15
ist.

## Claims

1. A device for defoaming blood, having a surface which comes into contact with blood and which is coated with a defoamer, **characterized in that** said defoamer is composed of a silicone compound with lipophobic microdomains containing fluorine.

2. The device according to claim 1, **characterized in that** the defoamer is prepared by reacting a silicone compound with a fluorinated dicarboxylic acid or with a fluorinated dicarboxylic acid derivative.

3. The device according to claim 2, **characterized in that** the fluorinated dicarboxylic acid derivative is tetrafluoro diethyl succinate.

4. The device according to any of claims 1 to 3, **characterized in that** a defoamer of the general formula:
{R'N-R-Si(CH₃)₂-[OSi(CH₃)₂]ₙ-R-(NR')-(CO)-(CF₂)ₖ-CO-}ₘ
is used, wherein
R is an alkylene or aralkyl group,
N is from 5 to 40, preferably 10 to 35,
R' is hydrogen, an alkyl or aralkyl group,
k is from 2 to 5 and
m is an integer between 1 and 15.

5. The device according to any of claims 1 to 4, **characterized in that** the surface to be coated with the defoamer comprises hydrophobic materials which are selected from the group comprising polyurethane, polyesters and other polymers.

6. The device according to any of claims 1 to 5, **characterized in that** it is designed as a filter which can be integrated into an extracorporeal circulation.

7. The device according to any of claims 1 to 5, **characterized in that** it is designed as defoaming filter in a blood reservoir.

8. Use of a defoamer-coated surface in an extracorporeal circulation, wherein said defoamer is composed of a silicone compound with lipophobic microdomains containing fluorine.

9. Defoamer, composed of a silicone-compound with lipophobic microdomains containing fluorine, wherein the microdomains contain fluorinated dicarboxylic acid or fluorinated dicarboxylic acid derivatives.

10. Defoamer according to claim 9, **characterized in that** the fluorinated dicarboxylic acid derivative is tetrafluoro diethyl succinate.

11. Defoamer according to any of claims 9 or 10, having the general formula
{R'N-R-Si(CH₃)₂-[OSi(CH₃)₂]ₙ-R-(NR')-(CO)-(CF₂)ₖ-CO-}ₘ
wherein
R is an alkylene or aralkylene group,
n is from 5 to 40, preferably 10 to 35,
R' is hydrogen, an alkyl or aralkyl group,
k is from 2 to 5 and
m is an integer between 1 and 15.

## Revendications

1. Dispositif de démoussage de sang avec une surface venant en contact avec le sang, qui est revêtue d'un agent antimousse, **caractérisé en ce que** l'agent antimousse est constitué d'un composé de silicone avec des microdomaines lipophobes contenant du fluor.

2. Dispositif selon la revendication 1, **caractérisé en ce que** l'agent antimousse est préparé en faisant réagir un composé de silicone avec un acide dicarboxylique fluoré ou avec un dérivé d'acide dicarboxylique fluoré.

3. Dispositif selon la revendication 2, **caractérisé en ce que** le dérivé d'acide dicarboxylique fluoré est le tétrafluorosuccinate de diéthyle.

4. Dispositif selon l'une des revendications 1 à 3, **caractérisé en ce que** l'on utilise un agent antimousse de la formule générale :
{R'N-R-Si(CH₃)₂-[OSi(CH₃)₂]ₙ-R-(NR')-(CO)-(CF₂)ₖ-CO-}ₘ
dans laquelle :
R est un groupe alkylène ou aralkylène,
n vaut de 5 à 40, de préférence de 10 à 35,
R' est un atome d'hydrogène, un groupe alkyle ou aralkyle, k vaut de 2 à 5, et
m est un nombre entier valant de 1 à 15.

5. Dispositif selon l'une des revendications 1 à 4, **caractérisé en ce que** la surface à revêtir avec l'agent antimousse comprend des matériaux hydrophobes choisis dans le groupe comprenant le polyuréthane, le polyester et d'autres polymères.

6. Dispositif selon l'une des revendications 1 à 5, **caractérisé en ce qu'**il est réalisé sous la forme d'un filtre insérable dans un circuit extracorporel.

7. Dispositif selon l'une des revendications 1 à 5, **caractérisé en ce qu'**il est réalisé sous la forme d'un filtre de démoussage dans un réservoir de sang.

8. Utilisation d'une surface revêtue d'un agent antimousse dans un circuit extracorporel, l'agent antimousse étant constitué d'un composé de silicone avec des microdomaines lipophobes contenant du fluor.

9. Agent antimousse constitué d'un composé de silicone avec des microdomaines lipophobes contenant du fluor, qui contiennent des acides dicarboxyliques fluorés ou des dérivés d'acides dicarboxyliques fluorés.

10. Agent antimousse selon la revendication 9, **caractérisé en ce que** le dérivé d'acide dicarboxylique fluoré est le tétrafluorosuccinate de diéthyle.

11. Agent antimousse selon la revendication 9 ou 10, ayant la formule générale
{R'N-R-Si(CH₃)₂-[OSi(CH₃)₂]ₙ-R-(NR')-(CO)-(CF₂)ₖ-CO-}ₘ
dans laquelle :
R est un groupe alkylène ou aralkylène,
N vaut de 5 à 40, de préférence de 10 à 35,
R' est un atome d'hydrogène, un groupe alkyle ou aralkyle, k vaut de 2 à 5, et
m est un nombre entier valant de 1 à 15.
